# FASCICULE DE BREVET EUROPEEN

(11) **EP 3 423 427 B1**
(45) Date de publication et mention de la délivrance du brevet: **01.01.2020**
(21) Numéro de dépôt: 17713724.7
(22) Date de dépôt: 03.03.2017
(51) Int. Cl.: C07C 17/383, C07C 21/18, C07C 21/22

(54) **COMPOSITION AZEOTROPIQUE OU QUASI-AZEOTROPIQUE COMPRENANT DE LA TRIFLUOROPROPYNE.**
AZEOTROPE ODER QUASIAZEOTROPE ZUSAMMENSETZUNG MIT TRIFLUORPROPYN
AZEOTROPIC OR QUASI-AZEOTROPIC COMPOSITION COMPRISING TRIFLUOROPROPYNE

(30) Priorité: 04.03.2016 FR 1651833
(43) Date de publication de la demande: 09.01.2019
(73) Titulaire: Arkema France, 92700 Colombes (FR)
(72) Inventeur: BABA-AHMED, Abdelatif, 69190 Saint-Fons (FR); COLLIER, Bertrand, 69230 Saint-Genis-Laval (FR); DEUR-BERT, Dominique, 69390 Charly (FR); WENDLINGER, Laurent, 69510 Soucieu En Jarrest (FR)
(74) Mandataire: Leca, François Michel
(86) Numéro de dépôt international: PCT/FR2017/050475
(87) Numéro de publication internationale: WO 2017/149255

(56) Documents cités:
- WO-A1-2013/088195

## Description

### Domaine technique de l'invention

La présente invention concerne une composition azéotropique comprenant de la trifluoropropyne. La présente invention concerne également un procédé de purification d'une composition comprenant de la trifluoropropyne et du 2,3,3,3-tetrafluoropropene.

### Arrière-plan technologique de l'invention

Les composés fluorés tels que les hydrofluorooléfines sont produits via des réactions de fluoration ou de dehydrofluoration d'hydrofluorocarbures, d'hydrochlorocarbures ou d'hydrochlorofluorocarbures. Ces réactions sont généralement mises en œuvre en présence d'acide fluorhydrique (HF) comme agent de fluoration. Par exemple, WO2013/088195 décrit la préparation de 2,3,3,3-tetrafluoropropene.

Au cours de ces réactions, de l'acide chlorhydrique est produit. Ce dernier peut être séparé des autres produits de la réaction par l'intermédiaire d'une colonne de distillation pour in fine récupérer l'acide chlorhydrique sans impuretés fluorées. En effet, l'acide chlorhydrique récupéré sous forme gazeuse est ensuite absorbé dans l'eau pour produire des solutions aqueuses d'acide chlorhydrique qui doivent avoir une faible teneur en produits fluorés.

Outre l'acide chlorhydrique, des impuretés organiques légères peuvent également se former au cours des réactions de fluoration. Notamment, lors de la production du 2,3,3,3-tetrafluoropropene (1234yf) les impuretés organiques légères sont par exemple la trifluoropropyne, le trifluorométhane, le pentafluoroéthane, le chloropentafluoroéthane ou le 1,1,1-trifluoroéthane. Ces impuretés organiques légères sont extraites du produit désiré lors de sa purification par l'intermédiaire d'une colonne de distillation dédiée à cet effet. La suppression de ces impuretés organiques légères est généralement accompagnée par une perte du produit désiré dont le point d'ébullition est souvent proche de celui des impuretés organiques légères. Ceci peut donc réduire le rendement global du procédé. Typiquement, la perte du produit désiré dû à la distillation intermédiaire des impuretés organiques légères est de l'ordre de 5%.

Il existe donc toujours un besoin pour mettre en œuvre un procédé de fluoration permettant de minimiser les pertes en produit désiré au cours de son processus de purification.

### Résumé de l'invention

Selon un premier aspect de la présente invention, une composition azéotropique ou quasi-azéotropique comprenant de l'acide chlorhydrique et de la trifluoropropyne est fournie.

Selon un mode de réalisation particulier, ladite composition azéotropique ou quasi-azéotropique comprend de 85% en poids à 99,999% en poids d'acide chlorhydrique et de 0,001% en poids à 15% en poids de trifluoropropyne sur base du poids total de la composition ; avantageusement ladite composition a un point d'ébullition de - 60°C à 0°C ; de préférence à une pression comprise entre 3 et 26 bara.

Selon un mode de réalisation préféré, ladite composition comprend également du pentafluoroéthane, du chloropentafluoroéthane ou de l'hexafluoroéthane.

Le demandeur a établi que la mise en œuvre des compositions azéotropiques ou quasi-azéotropiques ci-dessus facilite la purification du 2,3,3,3-tetrafluoropropene, en particulier favorise la séparation entre le 2,3,3,3-tetrafluoropropene et la trifluoropropyne qui ont des points d'ébullition relativement proches.

Selon un second aspect, l'invention fournit un procédé de séparation du 2,3,3,3-tetrafluoropropene et de la trifluoropropyne à partir d'une composition A comprenant du 2,3,3,3-tetrafluoropropene et de la trifluoropropyne, ledit procédé comprenant les étapes de :
i) mise en contact de ladite composition **A** avec un composé inorganique pour former une composition **B,**
ii) distillation de la composition **B** pour former un premier flux **B1** comprenant de la trifluoropropyne et le composé inorganique ; et un second flux **B2** comprenant du 2,3,3,3-tetrafluoropropene ; ledit composé inorganique étant l'acide chlorhydrique.

Selon un mode de réalisation préféré, la trifluoropropyne et le composé inorganique forme une composition azéotropique ou quasi-azéotropique.

Selon un mode de réalisation préféré, la quantité de trifluoropropyne dans ledit second flux **B2** est inférieure à la quantité initiale de trifluoropropyne dans la composition **B** ; avantageusement la quantité en trifluoropropyne dans le second flux **B2** peut être inférieure à 10% de la quantité initiale de trifluoropropyne contenue dans la composition **B** ; de préférence ledit second flux **B2** comprend moins de 1000 ppm de trifluoropropyne, de manière plus préférentielle ledit second flux **B2** comprend moins de 500 ppm de trifluoropropyne, en particulier ledit second flux **B2** comprend moins de 100 ppm, plus particulièrement ledit second flux **B2** comprend moins de 50 ppm ; de manière privilégiée ledit second flux **B2** est dépourvu de trifluoropropyne.

Selon un troisième aspect, l'invention fournit un procédé de production de 2,3,3,3-tetrafluoropropene comprenant les étapes de :
A) fluoration en présence d'un catalyseur d'un composé de formule (I) CX(Y)₂-CX(Y)ₘ-CHₘXY dans laquelle X et Y représentent indépendamment un atome d'hydrogène, de fluor ou de chlore et m = 0 ou 1 ; et/ou fluoration catalytique en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br; Y est indépendamment les uns des autres H, Cl, F, I ou Br ; n est 1, 2 ou 3 ; et m est 0, 1 ou 2 ; et p est 0 ou 1 ;
B) récupération d'un flux **C** comprenant du 2,3,3,3-tetrafluoropropene, de l'acide chlorhydrique et de la trifluoropropyne ;
C) distillation du flux **C** récupéré à l'étape B) pour former un premier flux **D** comprenant de l'acide chlorhydrique et de la trifluoropropyne ; et un second flux **E** comprenant du 2,3,3,3-tetrafluoropropene.

Selon un mode de réalisation préféré, le premier flux **D** comprenant de l'acide chlorhydrique et de la trifluoropropyne est récupéré en tête de colonne de distillation.

Selon un mode de réalisation préféré, la quantité de trifluoropropyne dans ledit second flux **E** est inférieure à la quantité initiale de trifluoropropyne dans le flux **C** ; avantageusement la quantité en trifluoropropyne dans le second flux **E** peut être inférieure à 10% de la quantité initiale de trifluoropropyne contenue dans le flux **C** ; de préférence ledit second flux **E** comprend moins de 1000 ppm de trifluoropropyne, de manière plus préférentielle ledit second flux **E** comprend moins de 500 ppm de trifluoropropyne, en particulier ledit second flux **E** comprend moins de 100 ppm, plus particulièrement ledit second flux **E** comprend moins de 50 ppm ; de manière privilégiée ledit second flux **E** est dépourvu de trifluoropropyne..

Selon un mode de réalisation préféré, le premier flux **D** comprend une composition azéotropique ou quasi-azéotropique comprenant de 85% en poids à 99,999% en poids d'acide chlorhydrique et de 0,001% en poids à 15% en poids de trifluoropropyne sur base du poids total de la composition; avantageusement ladite composition a un point d'ébullition de -60°C à 0°C de préférence à une pression comprise entre 3 et 26 bara.

Selon un mode de réalisation préféré, le flux **C** récupéré à l'étape B) et le premier flux **D** comprennent également du pentafluoroéthane (F125), du chloropentafluoroéthane (F115) ou de l'hexafluoroéthane (F116).

De préférence, le flux **C** récupéré à l'étape B) et le premier flux **D** comprennent également du difluorométhane (32), du 1,1,1-trifluoroéthane (143a) et/ou de l'acide fluorhydrique.

Selon un mode de réalisation préféré, le flux **C** et le second flux **E** comprennent également du 1,1,1,2,2-pentafluoropropane, du 1,3,3,3-tetrafluoropropene et des impuretés ayant un point d'ébullition inférieur au point d'ébullition du 2,3,3,3-tetrafluoropropene ; ledit second flux **E** étant distillé pour former un flux **G** comprenant du 2,3,3,3-tetrafluoropropene, du 1,1,1,2,2-pentafluoropropane et du 1,3,3,3-tetrafluoropropene ; et un flux **H** comprenant les impuretés ayant un point d'ébullition inférieur au point d'ébullition du 2,3,3,3-tetrafluoropropene.

La mise en œuvre d'une composition azéotropique ou quasi-azéotropique de trifluoropropyne et d'acide chlorhydrique permet de purifier aisément le 2,3,3,3-tetrafluoropropene de la trifluoropropyne et de l'acide chlorhydrique produit au cours de la réaction de fluoration. Les pertes de 2,3,3,3-tetrafluoropropene sont ainsi minimisées en éliminant avec l'acide chlorhydrique au moins la trifluoropropyne qui possède un point d'ébullition relativement proche de celui du 2,3,3,3-tetrafluoropropene.

### Description détaillée de l'invention

L'invention est maintenant décrite plus en détail et de façon non limitative dans la description qui suit.

Selon un premier aspect, l'invention fournit une composition azéotropique ou quasi-azéotropique comprenant de l'acide chlorhydrique et de la trifluoropropyne.

Le terme « composition azéotropique » désigne un mélange liquide de deux ou plusieurs composés se comportant comme une substance unique, et qui bout à température fixe en gardant une composition en phase liquide identique à celle de la phase gaz. Le terme « composition quasi-azéotropique » désigne un mélange liquide de deux ou plusieurs composés ayant un point d'ébullition constant ou qui a tendance à ne pas se fractionner lorsqu'il est soumis à l'ébullition ou l'évaporation.

Selon un mode de réalisation préféré, la composition comprend de 85% en poids à 99,999% en poids d'acide chlorhydrique et de 0,001% en poids à 15% en poids de trifluoropropyne sur base du poids total de la composition. Avantageusement, la composition comprend de 87% en poids à 99,999% en poids d'acide chlorhydrique et de 0,001% en poids à 13% en poids de trifluoropropyne sur base du poids total de la composition. De préférence, la composition comprend de 88% en poids à 99,999% en poids d'acide chlorhydrique et de 0,001% en poids à 12% en poids de trifluoropropyne sur base du poids total de la composition. En particulier, la composition comprend de 90% en poids à 99,999% en poids d'acide chlorhydrique et de 0,001% en poids à 10% en poids de trifluoropropyne sur base du poids total de la composition.

Selon un mode de réalisation préféré, ladite composition a un point d'ébullition de -60°C à 0°C, avantageusement ladite composition a un point d'ébullition de -55°C à 0°C, de préférence ladite composition a un point d'ébullition de -50°C à -5°C.

Selon un mode de réalisation préféré, ladite composition a un point d'ébullition de -60°C à 0°C, avantageusement ladite composition a un point d'ébullition de -55°C à 0°C, de préférence ladite composition a un point d'ébullition de -50°C à -5°C à une pression de 3 à 26 bara.

Avantageusement, ladite composition a un point d'ébullition de -60°C à 0°C, avantageusement ladite composition a un point d'ébullition de -55°C à 0°C, de préférence ladite composition a un point d'ébullition de -50°C à -5°C à une pression de 4 à 26 bara.

De préférence, ladite composition a un point d'ébullition de - 60°C à 0°C, avantageusement ladite composition a un point d'ébullition de -55°C à 0°C, de préférence ladite composition a un point d'ébullition de -50°C à -5°C à une pression de 5 à 23 bara.

Selon un mode de réalisation préféré, ladite composition comprend également du pentafluoroéthane (F125) et/ou du chloropentafluoroéthane (F115) et/ou de l'hexafluoroéthane (F116). Avantageusement, la proportion totale du pentafluoroéthane (F125), du chloropentafluoroéthane (F115) ou de l'hexafluoroéthane (F116) dans ladite composition est inférieure à 2% en poids sur base du poids total de ladite composition. La proportion totale se rapporte à la somme des proportions individuelles en pentafluoroéthane (F125), en chloropentafluoroéthane (F115) et en hexafluoroéthane (F116). De préférence, la proportion totale du pentafluoroéthane (F125), chloropentafluoroéthane (F115) ou de l'hexafluoroéthane (F116) dans ladite composition est inférieure à 1% en poids sur base du poids total de ladite composition, en particulier inférieure à 0,5% en poids, plus particulièrement inférieure à 0,1% en poids sur base du poids total de ladite composition.

Selon un second aspect, la présente invention fournit un procédé de séparation du 2,3,3,3-tetrafluoropropene et de la trifluoropropyne à partir d'une composition **A** comprenant du 2,3,3,3-tetrafluoropropene et de la trifluoropropyne, ledit procédé comprenant les étapes de :
i) mise en contact de ladite composition **A** avec un composé inorganique pour former une composition **B,**
ii) purification, de préférence distillation, de la composition **B** pour former un premier flux **B1** comprenant de la trifluoropropyne et le composé inorganique ; et un second flux **B2** comprenant du 2,3,3,3-tetrafluoropropene.

Le composé inorganique peut être sous forme liquide ou sous forme gazeuse. De préférence, le premier flux **B1** comprenant la trifluoropropyne et le composé inorganique forme une composition azéotropique ou quasi-azéotropique. Le premier flux **B1** peut comprendre de 85% en poids à 99,999% en poids de composé inorganique et de 0,001% en poids à 15% en poids de trifluoropropyne sur base du poids total du premier flux **B1** ; avantageusement de 87% en poids à 99,999% en poids de composé inorganique et de 0,001% en poids à 13% en poids de trifluoropropyne ; de préférence de 88% en poids à 99,999% en poids de composé inorganique et de 0,001% en poids à 12% en poids de trifluoropropyne ; en particulier de 90% en poids à 99,999% en poids de composé inorganique et de 0,001% en poids à 10% en poids de trifluoropropyne.

Selon un mode de réalisation préféré, ledit composé inorganique est l'acide chlorhydrique. Ainsi, le premier flux **B1** peut comprendre une composition azéotropique ou quasi-azéotropique comprenant la trifluoropropyne et l'acide chlorhydrique. La formation privilégiée de la composition azéotropique ou quasi-azéotropique comprenant la trifluoropropyne et l'acide chlorhydrique facilite la séparation entre la trifluoropropyne et le 2,3,3,3-tetrafluoropropene contenu dans la composition **A.** Ainsi, le premier flux **B1** peut donc comprendre, sous forme azéotropique ou quasi-azéotropique, de 85% en poids à 99,999% en poids d'acide chlorhydrique et de 0,001% en poids à 15% en poids de trifluoropropyne sur base du poids total du premier flux **B1** ; avantageusement de 87% en poids à 99,999% en poids d'acide chlorhydrique et de 0,001% en poids à 13% en poids de trifluoropropyne ; de préférence, de 88% en poids à 99,999% en poids d'acide chlorhydrique et de 0,001% en poids à 12% en poids de trifluoropropyne ; en particulier, de 90% en poids à 99,999% en poids d'acide chlorhydrique et de 0,001% en poids à 10% en poids de trifluoropropyne.

Le premier flux **B1** peut avoir un point d'ébullition de -60°C à 0°C, avantageusement de -55°C à 0°C, de préférence de -50°C à -5°C à une pression de 3 à 26 bara. Avantageusement, le premier flux **B1** peut avoir un point d'ébullition de -60°C à 0°C, avantageusement de -55°C à 0°C, de préférence de -50°C à -5°C à une pression de 4 à 26 bara. De préférence, le premier flux **B1** peut avoir un point d'ébullition de -60°C à 0°C, avantageusement ladite composition a un point d'ébullition de -55°C à 0°C, de préférence ladite composition a un point d'ébullition de -50°C à - 5°C à une pression de 5 à 23 bara.

Le second flux **B2** comprenant le 2,3,3,3-tetrafluoropropene peut contenir une faible quantité en trifluoropropyne. La quantité en trifluoropropyne dans le second flux **B2** est inférieure à la quantité initiale de trifluoropropyne, exprimée en mole, dans la composition **B.** La quantité en trifluoropropyne dans le second flux **B2** peut être inférieure à 50% de la quantité initiale de trifluoropropyne contenue dans la composition **B.** Avantageusement, la quantité en trifluoropropyne dans le second flux **B2** peut être inférieure à 25% de la quantité initiale de trifluoropropyne contenue dans la composition **B.** De préférence, la quantité en trifluoropropyne dans le second flux **B2** peut être inférieure à 10% de la quantité initiale de trifluoropropyne contenue dans la composition **B.** En particulier, la quantité en trifluoropropyne dans le second flux **B2** peut être inférieure à 5% de la quantité initiale de trifluoropropyne contenue dans la composition **B.** Plus particulièrement, la quantité en trifluoropropyne dans le second flux **B2** peut être inférieure à 1% de la quantité initiale de trifluoropropyne contenue dans la composition **B.**

Selon un mode de réalisation préféré, ledit second flux **B2** comprend moins de 1000 ppm de trifluoropropyne ; avantageusement ledit second flux **B2** comprend moins de 500 ppm de trifluoropropyne ; de préférence ledit second flux **B2** comprend moins de 100 ppm de trifluoropropyne ; de manière plus préférentielle ledit second flux **B2** comprend moins de 50 ppm ; en particulier ledit second flux **B2** est dépourvu de trifluoropropyne. Le terme « dépourvu » tel qu'utilisé ici se réfère à une quantité de trifluoropropyne dans ledit second flux **B2** inférieure à 20 ppm, avantageusement inférieure à 10 ppm, de préférence inférieure à 1 ppm.

Selon un troisième aspect, l'invention fournit un procédé de production du 2,3,3,3-tetrafluoropropene. Ledit procédé comprend les étapes de :
A) fluoration en présence d'un catalyseur d'un composé de formule (I) CX(Y)₂-CX(Y)ₘ-CHₘXY dans laquelle X et Y représentent indépendamment un atome d'hydrogène, de fluor ou de chlore et m = 0 ou 1 ; et/ou fluoration en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;
B) récupération d'un flux C comprenant du 2,3,3,3-tetrafluoropropene, de l'acide chlorhydrique et de la trifluoropropyne ;
C) purification, de préférence distillation, du flux **C** récupéré à l'étape B) pour former un premier flux **D** comprenant de l'acide chlorhydrique et de la trifluoropropyne ; et un second flux **E** comprenant du 2,3,3,3-tetrafluoropropene.

Selon un mode de réalisation préféré, l'étape A) du présent procédé peut être effectuée en présence d'un catalyseur à partir d'un composé de formule (I) ou (II) sélectionné parmi le groupe consistant en 2-chloro-3,3,3-trifluoro-1-propene (HFCO-1233xf), 1,1,1,2,3-pentachloropropane (HCC-240db), 1,1,2,2,3-pentachloropropane (HCC-240aa), 2,3-dichloro-1,1,1-trifluoropropane (HCFC-243db), 1,1,2,3-tetrachloro-1-propene (HCO-1230xa), 2,3,3,3-tetrachloro-1-propene (HCO-1230xf), 2-chloro-1,1,1,2-tetrafluoropropane (HCFC-244bb), 1,1,1,3,3-pentachloropropane (HCC-240fa), 1,1,3,3-tetrachloropropene (HCO-1230za), 1,3,3,3-tetrachloropropène (HCO-1230zd), 1-chloro-3,3,3-trifluoropropene (HCFO-1233zd), 1,1,1,3-tetrachloropropane (HCC-250fb), 1,1,3-trichloropropene (HCO-1240za), 3,3,3-trichloropropene (HCO-1240zf). De préférence, l'étape A) du présent procédé peut être effectuée en présence d'un catalyseur à partir d'un composé de formule (I) ou (II) sélectionné parmi le groupe consistant en 2-chloro-3,3,3-trifluoro-1-propene (HFCO-1233xf), 1,1,1,2,3-pentachloropropane (HCC-240db), 1,1,2,2,3-pentachloropropane (HCC-240aa), 2,3-dichloro-1,1,1-trifluoropropane (HCFC-243db), 1,1,2,3-tetrachloro-1-propene (HCO-1230xa), 2,3,3,3-tetrachloro-1-propene (HCO-1230xf), 2-chloro-1,1,1,2-tetrafluoropropane (HCFC-244bb).

De préférence, la fluoration catalytique est réalisée en phase gazeuse.

Le catalyseur utilisé dans le présent procédé de production de 2,3,3,3-tetrafluoropropene peut être par exemple à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, I.1-5 et 28-32), au document EP 939071 (paragraphe [0022]), au document WO 2008/054781 (en p.9 I.22-p.10 I.34), et au document WO 2008/040969 (revendication 1), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de nickel.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, I.30-p.7 I.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF. Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h. Cette activation peut être suivie d'une étape d'activation de fluoration finale en présence d'un agent d'oxydation, d'HF et de composés organiques. Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :
- avec un rapport molaire HF / composé de formule (I) et/ou (II) de 3:1 à 150:1, de préférence de 4:1 à 125:1 et de manière plus particulièrement préférée de 5:1 à 100:1 ;
- avec un temps de contact de 3 à 100 s, de préférence 4 à 75 s et plus particulièrement 5 à 50 s (volume de catalyseur divisé par le flux entrant total, ajusté à la température et à la pression de fonctionnement) ;
- à une pression allant de la pression atmosphérique à 20 bar, de préférence de 2 à 18 bar et plus particulièrement de 3 à 15 bars;
- à une température (température du lit de catalyseur) de 200 à 450°C, de préférence de 250 à 400°C, et plus particulièrement de 280 à 380°C.

La durée de l'étape de réaction est typiquement de 10 à 8000 heures, de préférence de 50 à 5000 heures et de manière plus particulièrement préférée de 70 à 1000 heures.

Un agent oxydant, de préférence l'oxygène, peut éventuellement être ajouté lors de la réaction de fluoration. Le rapport molaire oxygène / composés organiques peut être de 0,005 à 2, de préférence de 0,01 à 1,5. L'oxygène peut être introduit pur ou sous forme d'air ou de mélange oxygène / azote. On peut également remplacer l'oxygène par du chlore.

De préférence, le premier flux **D** comprenant l'acide chlorhydrique et la trifluoropropyne est récupéré en tête de colonne de distillation. De préférence, le premier flux **D** est une composition azéotropique ou quasi-azéotropique d'acide chlorhydrique et de trifluoropropyne.

La formation d'une composition azéotropique ou quasi-azéotropique d'acide chlorhydrique et de trifluoropropyne permet d'améliorer la séparation entre la trifluoropropyne et le 2,3,3,3-tetrafluoropropene. Ainsi, le flux **E** comprenant le 2,3,3,3-tetrafluoropropene sera dépourvu de trifluoropropyne ou en contiendra des quantités suffisamment faibles pour faciliter sa purification ultérieure.

Selon un mode de réalisation préféré, le premier flux **D** peut comprendre, sous forme azéotropique ou quasi-azéotropique, de 85% en poids à 99,999% en poids d'acide chlorhydrique et de 0,001% en poids à 15% en poids de trifluoropropyne sur base du poids total du premier flux **D ;** avantageusement de 87% en poids à 99,999% en poids d'acide chlorhydrique et de 0,001% en poids à 13% en poids de trifluoropropyne ; de préférence de 88% en poids à 99,999% en poids d'acide chlorhydrique et de 0,001% en poids à 12% en poids de trifluoropropyne ; en particulier de 90% en poids à 99,999% en poids d'acide chlorhydrique et de 0,001% en poids à 10% en poids de trifluoropropyne.

Le premier flux **D** peut avoir un point d'ébullition de -60°C à 0°C, avantageusement de - 55°C à 0°C, de préférence de -50°C à -5°C à une pression de 3 à 26 bara. Avantageusement, le premier flux **D** peut avoir un point d'ébullition de -60°C à 0°C, avantageusement de -55°C à 0°C, de préférence de -50°C à -5°C à une pression de 4 à 26 bara. De préférence, le premier flux **D** peut avoir un point d'ébullition de -60°C à 0°C, avantageusement de -55°C à 0°C, de préférence de -50°C à -5°C à une pression de 5 à 23 bara.

De préférence, l'étape C) du présent procédé est effectuée dans des conditions opératoires aptes à permettre la formation du premier flux **D** comprenant de l'acide chlorhydrique et un ou plusieurs composés formant des azéotropes ou des quasi-azéotropes avec l'acide chlorhydrique. Outre l'azéotrope ou le quasi-azéotrope HCl/trifluoropropyne, l'étape C) peut être effectuée de sorte à récupérer dans ledit premier flux **D,** l'azéotrope ou le quasi-azéotrope HCl/pentafluoroéthane (F125), HCl/chloropentafluoroéthane (F115) et/ou HCl/hexafluoroéthane (F116). Ainsi, outre la trifluoropropyne, d'autres impuretés organiques légères peuvent être récupérées avec l'acide chlorhydrique. Ainsi, la distillation dédiée à l'élimination des impuretés organiques légères peut être supprimée ou au moins facilitée de sorte à minimiser les pertes en 2,3,3,3-tetrafluoropropene.

Ainsi, selon un mode de réalisation particulier, le flux C récupéré à l'étape B) et ledit premier flux **D** comprennent également du pentafluoroéthane (F125) et/ou du chloropentafluoroéthane (F115) et/ou de l'hexafluoroéthane (F116). Avantageusement, la proportion totale du pentafluoroéthane (F125), du chloropentafluoroéthane (F115) ou de l'hexafluoroéthane (F116) dans ledit premier flux **D** est inférieure à 2% en poids sur base du poids total de ladite composition. De préférence, la proportion totale du pentafluoroéthane (F125), du chloropentafluoroéthane (F115) ou de l'hexafluoroéthane (F116) dans ledit premier flux **D** est inférieure à 1% en poids sur base du poids total dudit premier flux **D,** en particulier inférieure à 0,5% en poids, plus particulièrement inférieure à 0,1% en poids.

Selon un mode de réalisation particulier, le flux C récupéré à l'étape B) et le premier flux **D** comprennent également du difluorométhane (F32) et/ou du 1,1,1-trifluoroéthane (F143a) et/ou de l'acide fluorhydrique. Avantageusement, la proportion totale en difluorométhane et/ou en 1,1,1-trifluoroéthane et/ou de l'acide fluorhydrique est inférieure à 0,5% en poids sur base du poids total du premier flux **D.**

Selon un mode de réalisation particulier, le premier flux **D** peut être purifié pour former une composition comprenant de l'acide chlorhydrique dans laquelle la teneur en trifluoropropyne est inférieure à 20 ppm, avantageusement inférieure à 10 ppm, de préférence inférieure à 1 ppm. La purification dudit premier flux **D** peut être effectuée par la mise en œuvre d'une étape d'hydrolyse catalytique ; de lavage par une solution acide ; et/ou une ou plusieurs étapes d'adsorption d'impuretés par un adsorbant.

De préférence, l'étape d'hydrolyse catalytique peut être effectuée par la mise en contact dudit premier flux **D** avec un lit catalytique, qui de préférence est un lit de charbon actif, en présence d'eau. La température de l'étape d'hydrolyse catalytique est de préférence de 100 à 200°C, notamment de 120 à 170°C, et plus particulièrement de 130 à 150°C. La pression est de préférence de 0,5 à 3 barg, notamment de 1 à 2 barg. Le temps de contact est de préférence de 1 s à 1 min, notamment de 2 s à 30 s, plus particulièrement de 4 s à 15 s, et tout particulièrement de 5 s à 10 s. La quantité d'eau dans ledit premier flux **D** soumis à l'hydrolyse catalytique est ajustée de sorte que le rapport molaire de l'eau par rapport à la somme des composés autre que l'acide chlorhydrique dans ledit premier flux **D** soit supérieur à 1, de préférence supérieur ou égal à 2, ou à 3, ou à 4, ou à 5, ou à 6 ou à 6,5. Un apport d'eau peut être prévu si nécessaire. L'étape d'hydrolyse catalytique peut être mise en œuvre pour hydrolyser des composés tels que COF₂, COFCl, CF₃COF si ceux-ci sont présents dans le flux C et le premier flux **D.** Suite à cette étape d'hydrolyse catalytique, un flux **F1** est récupéré. Ce flux **F1** comprend, outre l'acide chlorhydrique, de la trifluoropropyne et éventuellement du pentafluoroéthane (F125), du chloropentafluoroéthane (F115), de l'hexafluoroéthane (F116), du difluorométhane (F32), du 1,1,1-trifluoroéthane (F143a) et/ou de l'acide fluorhydrique et optionnellement de l'acide trifluoroacétique issu de l'hydrolyse de CF₃COF. L'étape de lavage par une solution acide peut être effectuée dans une colonne à plateaux, telle qu'une colonne à plateaux perforés, ou à cloches, ou à clapets ou de type Dualflow®. Il peut également s'agir d'une colonne à garnissage. Le lavage du flux gazeux **F1** est effectué de préférence à contre-courant : le flux gazeux **F1** est alimenté en pied, et une solution acide est alimentée en tête de colonne. A titre de solution acide, on peut notamment utiliser une solution d'HCl, à une concentration massique pouvant aller par exemple de 5 à 60 %, notamment de 10 à 50 %, plus préférentiellement de 20 à 45 % et en particulier de 30 à 35 %. Le lavage par la solution acide est de préférence mis en œuvre à une température de 5 à 50°C, et plus particulièrement de 7 à 40°C ; et/ou à une pression de 0,1 à 4 barg, de préférence de 0,3 à 2 barg, plus préférentiellement de 0,5 à 1,5 barg. Un ajout d'acide borique au stade du lavage par la solution acide peut également être effectué afin de complexer les ions fluorures. Par exemple, l'ajout de 2000 à 8000 ppm de H₃BO₃ permet d'améliorer l'élimination de certains composés fluorés. Le flux gazeux lavé **F2** issu de l'étape de lavage peut être soumis à une étape d'adsorption sur un lit de charbon actif pour former un flux **F3.** Les impuretés adsorbées par le lit de charbon actif sont en premier lieu la trifluoropropyne et éventuellement le pentafluoroéthane (F125), le chloropentafluoroéthane (F115), l'hexafluoroéthane (F116), le difluorométhane (F32) ou le 1,1,1-trifluoroéthane (F143a). L'étape d'adsorption sur lit de charbon actif peut être mise en œuvre dans des gammes de pression et de température qui ont déjà été indiquées ci-dessus en relation avec l'étape de lavage par une solution acide. Le flux gazeux purifié **F3** est soumis à une étape d'absorption adiabatique ou isotherme permettant d'absorber l'acide chlorhydrique du flux gazeux **F3** dans une solution aqueuse pour former une solution aqueuse d'acide chlorhydrique **F4**. Cette solution aqueuse peut être simplement de l'eau déminéralisée, ou alternativement peut être une solution acide. Généralement, cette étape d'absorption est effectuée sur une colonne de mise en contact à contre-courant, la solution aqueuse étant fournie en tête et le flux gazeux en pied. La réaction d'absorption de l'acide chlorhydrique dans l'eau étant exothermique, il est préférable de limiter la pression à laquelle cette opération est conduite. En général, la pression est inférieure à 2 barg et de préférence inférieure à 1,5 barg. De cette façon la température d'absorption n'excède pas 130°C, et de préférence 120°C. Pour résister à la corrosion, la colonne peut être en graphite ou bien en acier revêtu de polytétrafluoroéthylène (PTFE). Les internes de colonne peuvent être par exemple soit en graphite soit en polyfluorure de vinylidène (PVDF). Un flux gazeux désacidifié **F5** est récolté en tête. Ce flux peut être soit rejeté à l'atmosphère via une colonne de sécurité de neutralisation, soit envoyé vers un incinérateur. Une solution d'acide chlorhydrique **F4** est récoltée en pied. La concentration massique d'acide chlorhydrique dans la solution **F4** peut être de 5 à 50 %, de préférence de 15 à 40 %, et plus particulièrement de 30 à 35 %. Si la pureté de la solution d'acide chlorhydrique **F4** collectée n'est pas suffisante, et notamment si la teneur en HF reste supérieure au seuil souhaité, il est possible de procéder à une autre étape de traitement, à savoir une étape d'adsorption sur un gel de silice. La température de la solution d'acide chlorhydrique **F4** doit être aussi faible que possible, et par exemple inférieure ou égale à 35°C, car l'adsorption sur le gel de silice est exothermique. Au-dessus de cette température, l'efficacité d'adsorption diminue fortement. Le temps de contact est compris entre quelques minutes et quelques heures (de préférence entre 10 et 60 min). Les vitesses de passage sont lentes et comprises entre 1 et 20 m/h et de préférence entre 3 et 10 m/h. La pression de fonctionnement est de quelques bars (de 1 à 7 barg et de préférence de 1 à 5 barg). Le gel de silice comporte typiquement une taille de pores de 50 Å, alors que les gels classiques ont en général des tailles de pores de 20 Å au maximum. La teneur en fluorures de la solution d'HCl en entrée est de préférence inférieure ou égale à 100 ppm pour éviter tout risque de dégradation du gel de silice.

Selon un mode de réalisation préféré, le second flux **E** récupéré à l'étape C) du présent procédé comprenant le 2,3,3,3-tetrafluoropropene peut contenir une faible quantité de trifluoropropyne. De préférence, la quantité en trifluoropropyne dans le second flux **E** peut être inférieure à 10% de la quantité initiale de trifluoropropyne contenue dans le flux **C.** En particulier, la quantité en trifluoropropyne dans le second flux **E** peut être inférieure à 5% de la quantité initiale de trifluoropropyne contenue dans le flux **C.** Plus particulièrement, la quantité en trifluoropropyne dans le second flux **E** peut être inférieure à 1% de la quantité initiale de trifluoropropyne contenue dans le flux **C.**

Selon un mode de réalisation préféré, ledit second flux **E** comprend moins de 1000 ppm de trifluoropropyne ; avantageusement ledit second flux **E** comprend moins de 500 ppm de trifluoropropyne ; de préférence ledit second flux **E** comprend moins de 100 ppm de trifluoropropyne ; en particulier ledit second flux **E** est dépourvu de trifluoropropyne. Le terme « dépourvu » tel qu'utilisé ici se réfère à une quantité de trifluoropropyne dans ledit second flux **E** inférieure à 20 ppm, avantageusement inférieure à 10 ppm, de préférence inférieure à 1 ppm.

Selon un mode de réalisation préféré, le flux **C** et le second flux **E** comprennent également du 1,1,1,2,2-pentafluoropropane (245cb), du 1,3,3,3-tetrafluoropropene (1234ze) et des impuretés ayant un point d'ébullition inférieur au point d'ébullition du 2,3,3,3-tetrafluoropropène ; ledit second flux **E** étant distillé pour former un flux **G** comprenant du 2,3,3,3-tetrafluoropropène (1234yf), du 1,1,1,2,2-pentafluoropropane (245cb) et du 1,3,3,3-tetrafluoropropene (1234ze) ; et un flux **H** comprenant les impuretés ayant un point d'ébullition inférieur au point d'ébullition du 2,3,3,3-tetrafluoropropène. Les impuretés ayant un point d'ébullition inférieur au point d'ébullition du 2,3,3,3-tetrafluoropropène peuvent être le trifluorométhane (F23) et/ou le monofluorométhane (F41). En outre, le second flux **E** peut comprendre du difluorométhane (F32), du pentafluoroéthane (F125), du 1,1,1-trifluoroéthane (F143a), du trifluoropropyne ou du 1-chloro-pentafluoroéthane (F115) en très faibles quantités. Ainsi, le second flux **E** comprend moins de 10 ppm de difluorométhane (F32), moins de 10 ppm de pentafluoroéthane (F125), moins de 10 ppm de 1,1,1-trifluoroéthane (F143a), moins de 10 ppm de trifluoropropyne et/ou moins de 10 ppm de 1-chloro-pentafluoroéthane (F115).

Selon un mode de réalisation préféré, le flux **G** comprenant du 2,3,3,3-tetrafluoropropene, du 1,1,1,2,2-pentafluoropropane et du 1,3,3,3-tetrafluoropropene contient moins de 1 ppm de difluorométhane (F32), moins de 1 ppm de pentafluoroéthane (F125), moins de 1 ppm de 1,1,1-trifluoroéthane (F143a), moins de 1 ppm de trifluoropropyne et/ou moins de 1 ppm de 1-chloro-pentafluoroéthane (F115), si le second flux **E** contenait l'un de ces composés.

Le demandeur a ainsi mis en évidence que l'élimination combinée d'acide chlorhydrique et de la trifluoropropyne permet de minimiser les pertes en 2,3,3,3-tetrafluoropropene en supprimant ou en facilitant la distillation dédiée à l'élimination des impuretés organiques légères compte tenu des faibles teneurs en impuretés organiques légères contenue dans le second flux **E.**

### Exemple

La formation de l'azéotrope ou du quasi-azéotrope acide chlorhydrique / trifluoropropyne a été simulée avec le logiciel Aspen. Les résultats sont présentés dans le tableau 1 ci-dessous.

**Tableau 1**

| Azéotrope HCl-Trifluoropropyne | | | |
|---|---|---|---|
| Température | Pression | Composition Azéotropique | |
| °C | bara | % poids HCl | % poids Trifluoropropyne |
| -60 | 3,72 | 93 | 7 |
| -55 | 4,33 | 94 | 6 |
| -50 | 5,29 | 95 | 5 |
| -45 | 6,4 | 97 | 3 |
| -42 | 7,15 | 97,5 | 2,5 |
| -41 | 7,41 | 97,5 | 2,5 |
| -40 | 7,69 | 98,5 | 1,5 |
| -39 | 7,96 | 98,5 | 1,5 |
| -38 | 8,25 | 99 | 1 |
| -37 | 8,54 | 99 | 1 |

## Revendications

1. Composition azéotropique ou quasi-azéotropique comprenant de l'acide chlorhydrique et de la trifluoropropyne.

2. Composition selon la revendication 1 comprenant de 85% en poids à 99,999% en poids d'acide chlorhydrique et de 0,001% en poids à 15% en poids de trifluoropropyne sur base du poids total de la composition ; avantageusement ladite composition a un point d'ébullition de -60°C à 0°C de préférence à une pression de 3 à 26 bara.

3. Composition selon l'une quelconque des revendications précédentes **caractérisée en ce qu'**elle comprend également du pentafluoroéthane, chloropentafluoroéthane ou de l'hexafluoroéthane.

4. Procédé de séparation du 2,3,3,3-tetrafluoropropene et de la trifluoropropyne à partir d'une composition **A** comprenant du 2,3,3,3-tetrafluoropropene et de la trifluoropropyne, ledit procédé comprenant les étapes de :
i) mise en contact de ladite composition **A** avec un composé inorganique pour former une composition **B** ;
ii) distillation de la composition **B** pour former un premier flux **B1** comprenant de la trifluoropropyne et le composé inorganique ; et un second flux **B2** comprenant du 2,3,3,3-tetrafluoropropene ;
ledit composé inorganique étant l'acide chlorhydrique.

5. Procédé selon la revendication précédente, **caractérisé en ce que** ledit premier flux **B1** comprenant la trifluoropropyne et le composé inorganique forme une composition azéotropique ou quasi-azéotropique.

6. Procédé selon l'une quelconque des revendication 4 ou 5 **caractérisé en ce que** la quantité de trifluoropropyne dudit second flux **B2** est inférieure à la quantité initiale de trifluoropropyne dans la composition **B** ; avantageusement la quantité en trifluoropropyne dans le second flux **B2** peut être inférieure à 10% de la quantité initiale de trifluoropropyne contenue dans la composition **B** ; de préférence ledit second flux **B2** comprend moins de 1000 ppm de trifluoropropyne, de manière plus préférentielle ledit second flux **B2** comprend moins de 500 ppm de trifluoropropyne, en particulier ledit second flux **B2** comprend moins de 100 ppm, plus particulièrement ledit second flux **B2** comprend moins de 50 ppm ; de manière privilégiée ledit second flux **B2** est dépourvu de trifluoropropyne.

7. Procédé de production du 2,3,3,3-tetrafluoropropene comprenant les étapes de :
A) fluoration en présence d'un catalyseur d'un composé de formule (I) CX(Y)₂-CX(Y)ₘ-CHₘXY dans laquelle X et Y représentent indépendamment un atome d'hydrogène, de fluor ou de chlore et m = 0 ou 1 ; et/ou fluoration en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;
B) récupération d'un flux **C** comprenant du 2,3,3,3-tetrafluoropropene, de l'acide chlorhydrique et de la trifluoropropyne ;
C) distillation du flux **C** récupéré à l'étape B) pour former un premier flux **D** comprenant de l'acide chlorhydrique et de la trifluoropropyne ; et un second flux **E** comprenant du 2,3,3,3-tetrafluoropropene.

8. Procédé selon la revendication précédente **caractérisé en ce que** le premier flux **D** comprenant de l'acide chlorhydrique et de la trifluoropropyne est récupéré en tête de colonne de distillation.

9. Procédé selon l'une quelconque des revendications précédentes 7 ou 8 **caractérisé en ce que** la quantité de trifluoropropyne dudit second flux **E** est inférieure à la quantité initiale de trifluoropropyne dans le flux **C** ; avantageusement la quantité en trifluoropropyne dans le second flux **E** peut être inférieure à 10% de la quantité initiale de trifluoropropyne contenue dans le flux **C** ; de préférence ledit second flux E comprend moins de 1000 ppm de trifluoropropyne ; de manière plus préférentielle ledit second flux **E** comprend moins de 500 ppm de trifluoropropyne ; en particulier ledit second flux **E** comprend moins de 100 ppm ; plus particulièrement ledit second flux **E** comprend moins de 50 ppm ; de manière privilégiée ledit second flux **E** est dépourvu de trifluoropropyne.

10. Procédé selon l'une quelconque des revendications 7 à 9 **caractérisé en ce que** le premier flux **D** comprend une composition azéotropique ou quasi-azéotropique comprenant de 85% en poids à 99,999% en poids d'acide chlorhydrique et de 0,001% en poids à 15% en poids de trifluoropropyne sur base du poids total du premier flux **D**; avantageusement ladite composition a un point d'ébullition de -60°C à 0°C de préférence à une pression comprise entre 3 et 26 bara.

11. Procédé selon l'une quelconque des revendications 7 à 10 **caractérisé en ce que** le flux **C** récupéré à l'étape B) et le premier flux **D** comprennent également du pentafluoroéthane (F125), du chloropentafluoroéthane (F115) ou de l'hexafluoroéthane (F116).

12. Procédé selon l'une quelconque des revendications 7 à 11 **caractérisé en ce que** le flux **C** récupéré à l'étape B) et le premier flux **D** comprennent également du difluorométhane (32), du 1,1,1-trifluoroéthane (143a) et/ou de l'acide fluorhydrique.

13. Procédé selon l'une quelconque des revendications 7 à 12 **caractérisé en ce que** le flux **C** et le second flux **E** comprennent également du 1,1,1,2,2-pentafluoropropane, du 1,3,3,3-tetrafluoropropene et des impuretés ayant un point d'ébullition inférieur au point d'ébullition du 2,3,3,3-tetrafluoropropène ; ledit second flux **E** étant distillé pour former un flux **G** comprenant du 2,3,3,3-tetrafluoropropène, du 1,1,1,2,2-pentafluoropropane et du 1,3,3,3-tetrafluoropropene ; et un flux **H** comprenant les impuretés ayant un point d'ébullition inférieur au point d'ébullition du 2,3,3,3-tetrafluoropropène.

## Patentansprüche

1. Azeotrope oder quasi-azeotrope Zusammensetzung, die Salzsäure und Trifluorpropin umfasst.

2. Zusammensetzung nach Anspruch 1, die 85 Gew.-% bis 99,999 Gew.-% Salzsäure und 0,001 Gew.-% bis 15 Gew.-% Trifluorpropin, bezogen auf das Gesamtgewicht der Zusammensetzung, umfasst, wobei vorteilhafterweise die Zusammensetzung einen Siedepunkt von -60°C bis 0°C vorzugsweise bei einem Druck von 3 bis 26 bar absolut aufweist.

3. Zusammensetzung nach einem der vorhergehenden Ansprüche, **dadurch gekennzeichnet, dass** sie außerdem Pentafluorethan, Chlorpentafluorethan oder Hexafluorethan umfasst.

4. Verfahren zur Trennung von 2,3,3,3-Tetrafluorpropen und Trifluorpropin ausgehend von einer Zusammensetzung **A,** die 2,3,3,3-Tetrafluorpropen und Trifluorpropin umfasst, wobei das Verfahren die folgenden Schritte umfasst:
i) Inkontaktbringen der Zusammensetzung **A** mit einer anorganischen Verbindung zur Herstellung einer Zusammensetzung **B;**
ii) Destillation der Zusammensetzung **B** zur Herstellung eines ersten Stroms **B1,** der Trifluorpropin und die anorganische Verbindung umfasst, und eines zweiten Stroms **B2**, der 2,3,3,3-Tetrafluorpropen umfasst,
wobei die anorganische Verbindung Salzsäure ist.

5. Verfahren nach dem vorhergehenden Anspruch, **dadurch gekennzeichnet, dass** der erste Strom **B1,** der Trifluorpropin und die anorganische Verbindung umfasst, eine azeotrope oder quasi-azeotrope Zusammensetzung bildet.

6. Verfahren nach einem der Ansprüche 4 oder 5, **dadurch gekennzeichnet, dass** die Menge an Trifluorpropin des zweiten Stroms **B2** kleiner als die Ausgangsmenge an Trifluorpropin in der Zusammensetzung **B** ist, wobei vorteilhafterweise die Menge an Trifluorpropin im zweiten Strom **B2** weniger als 10% der in der Zusammensetzung **B** enthaltenen Ausgangsmenge an Trifluorpropin betragen kann, wobei vorzugsweise der zweite Strom **B2** weniger als 1000 ppm Trifluorpropin umfasst, stärker bevorzugt der zweite Strom **B2** weniger als 500 ppm Trifluorpropin umfasst, insbesondere der zweite Strom **B2** weniger als 100 ppm umfasst, ganz besonders der zweite Strom **B2** weniger als 50 ppm umfasst, bevorzugt der zweite Strom **B2** frei von Trifluorpropin ist.

7. Verfahren zur Herstellung von 2,3,3,3-Tetrafluorpropen, umfassend die folgenden Schritte:
A) Fluorierung, in Gegenwart eines Katalysators, einer Verbindung der Formel (I) CX(Y)₂-CX(Y)ₘ-CHₘXY, worin X und Y unabhängig ein Wasserstoff-, Fluor- oder Chloratom darstellen und m = 0 oder 1; und/oder Fluorierung, in Gegenwart eines Katalysators, einer Verbindung der Formel (CXₙY₃₋ₙ)CHₚX₁₋ₚCHₘX₂₋ₘ (II), worin X unabhängig voneinander Cl, F, I oder Br ist; Y unabhängig voneinander H, Cl, F, I oder Br ist; n 1, 2 oder 3 ist; und m 0, 1 oder 2 ist; und p 0 oder 1 ist;
B) Gewinnen eines Stroms **C,** der 2,3,3,3-Tetrafluorpropen, Salzsäure und Trifluorpropin umfasst;
C) Destillation des im Schritt B) gewonnenen Stroms **C** zur Herstellung eines ersten Stroms **D,** der Salzsäure und Trifluorpropin umfasst, und eines zweiten Stroms **E,** der 2,3,3,3-Tetrafluorpropen umfasst.

8. Verfahren nach dem vorhergehenden Anspruch, **dadurch gekennzeichnet, dass** der erste Strom **D,** der Salzsäure und Trifluorpropin umfasst, am Kopf der Destillationskolonne gewonnen wird.

9. Verfahren nach einem der vorhergehenden Ansprüche 7 oder 8, **dadurch gekennzeichnet, dass** die Menge an Trifluorpropin des zweiten Stroms **E** kleiner als die Ausgangsmenge an Trifluorpropin im Strom **C** ist, wobei vorteilhafterweise die Menge an Trifluorpropin im zweiten Strom **E** weniger als 10% der im Strom **C** enthaltenen Ausgangsmenge an Trifluorpropin betragen kann, wobei vorzugsweise der zweite Strom **E** weniger als 1000 ppm Trifluorpropin umfasst, stärker bevorzugt der zweite Strom **E** weniger als 500 ppm Trifluorpropin umfasst, insbesondere der zweite Strom **E** weniger als 100 ppm umfasst, ganz besonders der zweite Strom **E** weniger als 50 ppm umfasst, bevorzugt der zweite Strom **E** frei von Trifluorpropin ist.

10. Verfahren nach einem der Ansprüche 7 bis 9, **dadurch gekennzeichnet, dass** der erste Strom **D** eine azeotrope oder quasi-azeotrope Zusammensetzung umfasst, die 85 Gew.-% bis 99,999 Gew.-% Salzsäure und 0,001 Gew.-% bis 15 Gew.-% Trifluorpropin, bezogen auf das Gesamtgewicht des ersten Stroms **D,** umfasst, wobei vorteilhafterweise die Zusammensetzung einen Siedepunkt von -60°C bis 0°C vorzugsweise bei einem Druck zwischen 3 und 26 bar absolut aufweist.

11. Verfahren nach einem der Ansprüche 7 bis 10, **dadurch gekennzeichnet, dass** der im Schritt B) gewonnene Strom **C** und der erste Strom **D** außerdem Pentafluorethan (F125), Chlorpentafluorethan (F115) oder Hexafluorethan (F116) umfassen.

12. Verfahren nach einem der Ansprüche 7 bis 11, **dadurch gekennzeichnet, dass** der im Schritt B) gewonnene Strom **C** und der erste Strom **D** außerdem Difluormethan (32), 1,1,1-Trifluorethan (143a) und/oder Flusssäure umfassen.

13. Verfahren nach einem der Ansprüche 7 bis 12, **dadurch gekennzeichnet, dass** der Strom **C** und der zweite Strom **E** außerdem 1,1,1,2,2-Pentafluorpropan, 1,3,3,3-Tetrafluorpropen und Verunreinigungen umfassen, die einen Siedepunkt unterhalb des Siedepunkts von 2,3,3,3-Tetrafluorpropen besitzen, wobei der zweite Strom **E** destilliert wird, um einen Strom **G,** der 2,3,3,3-Tetrafluorpropen, 1,1,1,2,2-Pentafluorpropan und 1,3,3,3-Tetrafluorpropen umfasst, und einen Strom **H,** der die Verunreinigungen mit einem Siedepunkt unterhalb des Siedepunkts von 2,3,3,3-Tetrafluorpropen umfasst, herzustellen.

## Claims

1. Azeotropic or quasi-azeotropic composition comprising hydrochloric acid and trifluoropropyne.

2. Composition according to Claim 1, comprising from 85% by weight to 99.999% by weight of hydrochloric acid and from 0.001% by weight to 15% by weight of trifluoropropyne, based on the total weight of the composition; advantageously, said composition has a boiling point from -60°C to 0°C preferably at a pressure of 3 to 26 bara.

3. Composition according to either one of the preceding claims, **characterized in that** it also comprises pentafluoroethane, chloropentafluoroethane or hexafluoroethane.

4. Process for separating 2,3,3,3-tetrafluoropropene and trifluoropropyne from a composition **A** comprising 2,3,3,3-tetrafluoropropene and trifluoropropyne, said process comprising the steps of:
i) bringing said composition **A** into contact with an inorganic compound in order to form a composition **B;**
ii) distilling the composition **B** in order to form a first stream **B1** comprising trifluoropropyne and the inorganic compound; and a second stream **B2** comprising 2,3,3,3-tetrafluoropropene;
said inorganic compound being hydrochloric acid.

5. Process according to the preceding claim, **characterized in that** said first stream **B1** comprising trifluoropropyne and the inorganic compound forms an azeotropic or quasi-azeotropic composition.

6. Process according to either one of Claims 4 and 5, **characterized in that** the amount of trifluoropropyne of said second stream **B2** is less than the initial amount of trifluoropropyne in the composition **B;** advantageously, the amount of trifluoropropyne in the second stream **B2** may be less than 10% of the initial amount of trifluoropropyne contained in the composition **B;** preferably, said second stream **B2** comprises less than 1000 ppm of trifluoropropyne, more preferentially, said second stream **B2** comprises less than 500 ppm of trifluoropropyne, in particular, said second stream **B2** comprises less than 100 ppm, more particularly, said second stream **B2** comprises less than 50 ppm; with particular preference, said second stream **B2** is devoid of trifluoropropyne.

7. Process for producing 2,3,3,3-tetrafluoropropene, comprising the steps of:
A) fluorination in the presence of a catalyst for a compound of formula (I) CX(Y)₂-CX(Y)ₘ-CHₘXY in which X and Y independently represent a hydrogen, fluorine or chlorine atom and m = 0 or 1; and/or fluorination in the presence of a catalyst for a compound of formula (CXₙY₃₋ₙ)CHₚX₁₋ₚCHₘX₂₋ₘ (II) in which X is, independently of each other, Cl, F, I or Br; Y is, independently of each other, H, Cl, F, I or Br; n is 1, 2 or 3; and m is 0, 1 or 2; and p is 0 or 1;
B) recovery of a stream **C** comprising 2,3,3,3-tetrafluoropropene, hydrochloric acid and trifluoropropyne;
C) distilling the stream **C** recovered in step B) in order to form a first stream **D** comprising hydrochloric acid and trifluoropropyne; and a second stream **E** comprising 2,3,3,3-tetrafluoropropene.

8. Process according to the preceding claim, **characterized in that** the first stream **D** comprising hydrochloric acid and trifluoropropyne is recovered at the top of the distillation column.

9. Process according to either one of the preceding Claims 7 and 8, **characterized in that** the amount of trifluoropropyne of said second stream **E** is less than the initial amount of trifluoropropyne in the stream **C;** advantageously, the amount of trifluoropropyne in the second stream **E** may be less than 10% of the initial amount of trifluoropropyne contained in the stream **C;** preferably, said second stream **E** comprises less than 1000 ppm of trifluoropropyne; more preferentially, said second stream **E** comprises less than 500 ppm of trifluoropropyne; in particular, said second stream **E** comprises less than 100 ppm; more particularly, said second stream **E** comprises less than 50 ppm; with particular preference, said second stream **E** is devoid of trifluoropropyne.

10. Process according to any one of Claims 7 to 9, **characterized in that** the first stream **D** comprises an azeotropic or quasi-azeotropic composition comprising from 85% by weight to 99.999% by weight of hydrochloric acid and from 0.001% by weight to 15% by weight of trifluoropropyne based on the total weight of the first stream **D;** advantageously, said composition has a boiling point from -60°C to 0°C preferably at a pressure of between 3 and 26 bara.

11. Process according to any one of Claims 7 to 10, **characterized in that** the stream **C** recovered in step B) and the first stream **D** also comprise pentafluoroethane (F125), chloropentafluoroethane (F115) or hexafluoroethane (F116).

12. Process according to any one of Claims 7 to 11, **characterized in that** the stream **C** recovered in step B) and the first stream **D** also comprise difluoromethane (32), 1,1,1-trifluoroethane (143a) and/or hydrofluoric acid.

13. Process according to any one of Claims 7 to 12, **characterized in that** the stream **C** and the second stream **E** also comprise 1,1,1,2,2-pentafluoropropane, 1,3,3,3-tetrafluoropropene and impurities having a boiling point lower than the boiling point of 2,3,3,3-tetrafluoropropene; said second stream **E** being distilled in order to form a stream **G** comprising 2,3,3,3-tetrafluoropropene, 1,1,1,2,2-pentafluoropropane and 1,3,3,3-tetrafluoropropene; and a stream **H** comprising impurities having a boiling point lower than the boiling point of 2,3,3,3-tetrafluoropropene.
